(19) **Europäisches Patentamt**
**European Patent Office**
**Office européen des brevets**

(11) **EP 4 455 707 A1**

(12) **EUROPEAN PATENT APPLICATION**

(43) Date of publication:
**30.10.2024 Bulletin 2024/44**

(21) Application number: **23169723.6**

(22) Date of filing: **25.04.2023**

(51) International Patent Classification (IPC):
*G01R 33/56* (2006.01)    *A61B 5/055* (2006.01)
*G06T 7/10* (2017.01)

(52) Cooperative Patent Classification (CPC):
**G01R 33/5608; A61B 5/0044; A61B 5/055;**
**A61B 5/7264;** G06N 3/0464; G06N 3/0475;
G06N 3/084; G06N 3/094

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **SHARMA, Sumit**
  **Eindhoven (NL)**
• **P S, Viswanath**
  **Eindhoven (NL)**
• **HEGDE, Amruta Venkatraman**
  **5656AG Eindhoven (NL)**
• **SARASWATHY, Suja**
  **Eindhoven (NL)**
• **ADHIKARY, Dhruba**
  **Eindhoven (NL)**
• **ALI MATTATHODI, Razeem Ahmad**
  **Eindhoven (NL)**
• **NEELAVALLI, Jaladhar**
  **Eindhoven (NL)**
• **NARAYANAN, Prasad**
  **Eindhoven (NL)**
• **VS, Vineeth**
  **Eindhoven (NL)**

(74) Representative: **Philips Intellectual Property &**
**Standards**
**High Tech Campus 52**
**5656 AG Eindhoven (NL)**

(54) **SYSTEM AND RELATED METHOD FOR 3D MULTI-VIEW PLANNING FOR MR IMAGING**

(57)    System (FS) and related method for facilitating image acquisition in medical imaging, comprising an input interface (IN) for receiving, when the system is in use, an input image volume of an object in an examination region (ER) of an imaging apparatus (IA). The system may include a plane segmentor (PD) to segment for an imaging plane (E) in the volume (*V0*), to so obtain a segmentation result, the said segmentation result including the said imaging plane as a subset in the volume. The system may further include an output interface (OUT) for providing output data in relation to the said imaging plane for controlling a data acquisition operation of the imaging apparatus.

**FIG. 3**

EP 4 455 707 A1

**Description**

FIELD OF THE INVENTION

The invention relates to system for facilitating image acquisition in medical imaging, in particular in MRI or other tomographic imaging modalities, to a related method, to a method of training a machine learning model for use in such system, to a training system for training such a model, to a computer program element and to a computer readable medium.

BACKGROUND OF THE INVENTION

Magnetic resonance imaging (MRI) is a celebrated imaging modality as it allows non-invasively obtaining imagery of internal anatomies and/or pathophysiology, without harmful side effects to patients.

For example, cardiac MRI is a commonly used technique for diagnosing a wide range of morphological and functional disorders of the heart, such as pericardial diseases, congenital diseases, heart valve diseases, etc. Cardiac MR images allow clinicians to non-invasively visualize heart structures through the acquisition of, for example, several double oblique planes, oriented to the heart chambers and valves in 3D space.

Imaging for these cardiac views help assess anomalies, ensures efficient coverage of the relevant heart regions, and enables comparisons across multiple modalities, thus enhancing patient care and cardiovascular research.

During a conventional cardiac MRI acquisition, the patient lies in a supine position in the scanner's bore. Today, an imaging operator follows a multi-step approach by starting with a transverse stack and successive scans such as Vertical Long Axis (VLA), Horizontal Long Axis (HLA), Mid-ventricular Short Axis (PSA), 4CH, etc., are planned, and then scanned with recursive geometrical references for each next scan.

In order to generate standard cardiac views for example, the operator identifies anatomical landmarks and uses them to compute imaging planes for the various views required for the clinical task at hand. Since the planning procedure requires extensive anatomical knowledge and technical expertise, many operators find it difficult to plan these views in a time-efficient and a reproducible manner.

SUMMARY OF THE INVENTION

There may therefore be a need for improving operation of tomographic imaging, in particular of the MRI type.

An object of the present invention is achieved by the subject matter of the independent claims where further embodiments are incorporated in the dependent claims. It should be noted that the following described aspect of the invention equally applies to the said methods, to the imaging arrangements, to the training system, to the computer program element and to the computer readable medium.

According to a first aspect of the invention there is provided a system for facilitating image acquisition in medical imaging, comprising:

an input interface for receiving, when the system is in use, an input image volume of a at least a part of an object in an examination region of an imaging apparatus;

a plane segmentor capable to segment for at least one imaging plane in the volume, to so obtain a segmentation result, the said segmentation result including the said imaging plane as a subset in the volume; and

an output interface for providing output data in relation to the said imaging plane for controlling a data acquisition operation of the imaging apparatus.

In embodiments, the system comprises an imager control interface configured to process the output data into a control signal for controlling the data acquisition operation by the imaging apparatus in order to acquire data from which is obtainable an image of the object in respect of the said imaging plane.

In embodiments, the said output data includes a specification of the imaging plane as a subset.

In embodiments, the specification includes any one of: i) a set of coordinate points, ii) an equation describing the subset as a geometrical locus.

In embodiments, the system may comprise, or may interface with, a specifier capable to specify the segmented subset of the imaging plane in terms of the specification.

In embodiments, the specifier is capable to specify the subset of the imaging plane based on a geometrical plane fitting algorithm.

In embodiments, the plane segmentor is based on a trained machine learning model.

In embodiments, the machine learning model is capable of multi-scale processing at different resolution levels.

In embodiments, the machine learning model is of the artificial neural network type.

In embodiments, the imaging apparatus is of the magnetic resonance type.

In embodiments, the system is integrated in a planning module.

In embodiments, the system may include decomposer and a composer, the decomposer capable to decompose the volume into patches, and to feed the patches into the segmentor to obtain a partial segmentation result per patch, and the recomposer capable of recomposing the partial segmentation results into the said segmentation result.

In another aspect there is provided a medical imaging arrangement, comprising the system of any one of the preceding claims, and further comprising the imaging apparatus and or a memory on which is stored the machine learning model.

In another aspect there is provided a computer-implemented method of facilitating image acquisition in medical imaging; comprising:

receiving, when the system is in use, an input image volume of a at least a part of an object in an examination region of an imaging apparatus;
segmenting for at least one imaging plane in the volume, to so obtain a segmentation result, the said segmentation result including the said imaging plane as a subset in the volume; and
providing output data in relation to the said imaging plane for controlling a data acquisition operation of the imaging apparatus.

In another aspect there is provided a training system capable of training, based on training data, the machine learning model of the system of any one of the preceding embodiments or aspects, or of the system as used in the arrangement.

In another aspect there is provided a computer-implemented method of training, based on training data, the machine learning model as used in the system of any one of the above mentioned embodiments.

In yet another aspect there is provided a computer program element, which, when being executed by at least one processing unit, is adapted to cause the processing unit to perform any one of the above mentioned methods.

In another aspect there is provided at least one computer readable medium having stored thereon the program element, or having stored thereon the machine learning model.

In another aspect, there is provided a use of the trained machine learning model in segmenting for imaging planes.

The proposed system furnishes, optionally fully automated, and accelerated planning, such for cardiac imaging or other. The proposed system and related method is configure to identify different clinically relevant imaging planes (and thus, views) of the region of interest (such as the heart), whilst maintaining image quality and reducing the time taken for an imaging exam, such as a cardiac MRI exam.

Specifically, the proposed system and method allow fully automated ROI view -planning, such as planning a cardiac imaging plane. The system uses, in embodiments, 3D heart survey imagery, which is low-resolution imagery, as input in order to predict structurally and clinically relevant cardiac view planes. The imaging planes are computed without acquiring intermediate scans of landmarks. In embodiments, a machine learning model is used. Having such a system that automatically identifies some or all the essential cardiac imaging planes in preferably real time, allows a user, who may have no or little cardiac scanning experience or manual cardiac planning workflow knowledge, to be still able to do a cardiac scan quickly and efficiently.

The proposed system may allow for automated MRI scan planning as a one-step solution without reliance on prior identification of anatomical landmarks. Previous approaches have attempted to solve this planning problem as 3D landmark detection problem first. However, it has been found that this type of detouring via generating data for 3D landmarks is more time consuming, more error prone, as compared to segmenting for imaging plane(s) directly as envisaged herein.

For example, conventional cardiac MRI workflow had involved the acquisition of multiple oblique planes/views of the heart and subsequent processing which is highly operator-dependent and time-consuming. The complexity associated with variance across patients in even a normal heart anatomy makes it even more difficult for an average user to precisely identify the relevant views, thereby compromising accuracy, operational cost, and diagnostic quality in addition to the time spent. Identifying different cardiac view planes such as VLA Vertical long axis (VLA), 3 Chamber view (3CH), 4 Chamber view (4CH), Short Axis stack (SA), etc. is still a manual multi-step process, that includes an acquisition of double-oblique slices planned through the identified cardiac structural biomarkers/landmarks by a trained technologist or a physician.

The proposed system and method amount to a novel plane prediction model that can automatically prescribe the diagnostic views necessary for cardiac MR planning, given merely a low-resolution 3D cardiac MR survey image as input. The plane prediction problem as envisaged herein may be implemented as a constrained segmentation problem which predicts one or multiple masks for the associated planes.

Optionally, an analytical plane description (such as an equation or other specification) based on the predicted imaging plane (representable as a mask for example) using RANSAC, SVD, or other algorithms are envisaged herein.

The proposed method may be extended for any oblique view extraction, given a 3D image specifically in augmented reality applications for example. Thus, the proposed system and method may be used for any 3D plane prediction problem and application. The proposed method may be used beyond the medical fields, for imaging objects, as needed, preferably using 3D or 4D imaging modalities.

Whilst cardiac imaging is mainly envisaged herein, the principles disclosed herein are not so limited, and may be applied readily to the imaging of other organs, anatomies, tissues and groups thereof.

*"user/operator"* relates to a person, such as medical personnel or other, operating the imaging apparatus or overseeing the imaging procedure. In other words, the user is in general not the patient.

*"object"* is used herein in the general sense to include animate "objects" such as a human or animal patient, or anatomic parts thereof but also includes inanimate objects such as an item of baggage in security checks or a product in non-destructive testing. However, the proposed system will be discussed herein with main reference to the medical field, so we will be referring to the "object" as "the patient" or a part of the patient, such as an anatomy or organ, or group of anatomies or organs of the patient.

In general, the term *"machine learning"* includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of the model is called "training". In general task performance by the ML module may improve measurably, with training experience. Training experience may include suitable training data a(suitably varied) and exposure of the model to such data. Task performance may improve the better the data represents the task to be learned. *"Training experience helps improve performance if the training data well represents a distribution of examples over which the final system performance is measured"*. The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 1.2.1, McGraw-Hill, 1997.

*"ROI"* or region of interest, such as the heart in cardiac imaging, is the anatomy of interest and the purpose of the intended imaging (task). The ROI may include an organ, an anatomy, tissue, or a group of one or more of the foregoing, or a respective part thereof.

*"landmark"* or anatomical landmark includes anatomical features that do not represent the clinical/medical purpose of the intended imaging task.

*"segmentation"* as understood herein is any computation arrangement or image processing operation that allows defining a subset of spatial points within a given 3D volume, although the principles herein may also be used in 4D or 2D sets. The segmentation herein does not rely on pre-existing contours of the structure (in the case, of the imaging plane) to be segmented for.

*"imaging plane"* as used herein is a subset of the input surview image volume and defines the view of tomographic/sectional view of the ROI that is sought in the imaging,

BRIEF DESCRIPTION OF THE DRAWINGS

Exemplary embodiments of the invention will now be described with reference to the following drawings, which, unless stated otherwise, are not to scale, wherein:

Fig. 1 shows a block diagram of a medical imaging arrangement;
Fig. 2 shows a block diagram of a facilitator system that may be used in data acquisition by the imaging apparatus;
Fig. 3 shows a block diagram of the facilitator system for computing an imaging plane;
Fig. 4 show, in perspective view, illustration of imaging planes so computed;
Fig. 5 illustrates imaging planes so computed in a different view that that of Fig. 4;
Fig. 6 shows a schematic block diagram of a machine learning model of the neural network type as may be used herein in embodiments;
Fig. 6A shows a block diagram of a machine learning model capable of multi-scale processing;
Fig. 7 shows a block diagram of a training system for training, based on training data, a machine learning model;
Fig. 8 shows a flow chart of a method of facilitating data acquisition for medical imaging; and
Fig. 9 shows a flow chart of a method of training, based on training data, a machine learning model.

DETAILED DESCRIPTION OF EMBODIMENTS

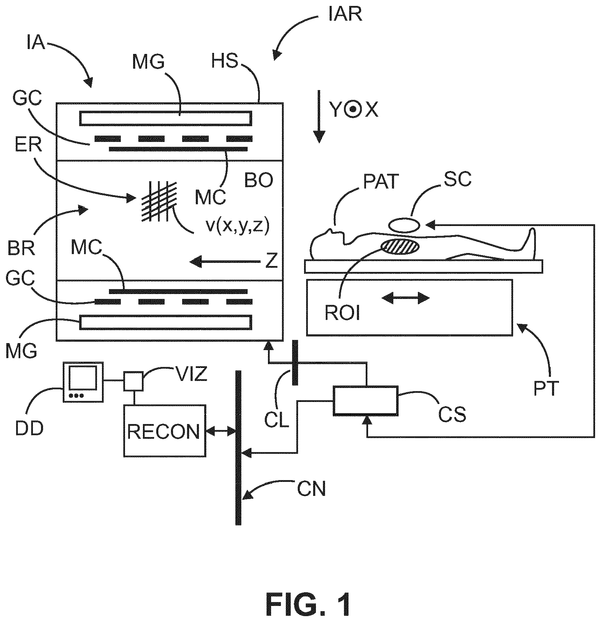

Referring first to Fig. 1, this shows a schematic block diagram of an imaging arrangement IAR as envisaged herein in embodiments. The imaging arrangement IAR includes an imaging apparatus IA ("imager") of the tomographic type suitable to obtain volumetric of sectional imagery of a patient PAT, For example, the imager IA may be an MRI (magnetic resonance imaging) apparatus IA configured to acquire MR medical images of human or animal patient PAT. The MRI imaging apparatus may be combined into a tandem/hybrid imaging or therapy system. Such tandem or hybrid imaging systems include MRI-PET(positron emission tomography) /SPECT (single-photon emission computed tomography) systems or CT(computed tomography)-PET/SPECT systems or indeed CT-MR systems, or in any other combination. Hybrid therapy-imaging systems include MRI-LINAC (linear accelerator) or MRI-Hyperthemia systems, or others still. However, such hybrid/tandem system are not required herein in all embodiments. Standalone MRI or CT or emission imagers IA are also envisaged.

The magnetic resonance imaging ("MRI") scanner IA may include a housing HS defining a generally cylindrical scanner bore BR inside of which an associated imaging subject PAT is disposed. Main magnetic field coils MG are disposed inside the housing HS. Main magnetic field coils may be generally of solenoidal configuration to produce a main Bo magnetic field directed along a Z-direction lying parallel to a central axis of the scanner bore BR. The main magnetic field coils are typically superconducting coils disposed inside in cryo-shrouding, although resistive main magnets can also be used. Particularly at higher field strengths and therefore higher frequencies, superconducting magnets are preferred. Whilst Fig. 1 shows a closed cylindrical design, this is not a requirement herein as open design MRI scanners with U-shaped magnets are also envisaged herein, although the principles of what is proposed herein will be of particular benefit to closed design MRI scanners due to their restricted patient space as will become apparent below.

The housing HS may also house or support magnetic field gradient coils GC for selectively producing magnetic field gradients along the Z-direction, along in-plane directions transverse to the Z-direction (such as along Cartesian X- and Y-directions), or along other selected directions.

The housing HS also houses or supports radio frequency head or body coils MC (referred to herein as main coils) for selectively exciting and/or detecting magnetic resonances. Although birdcage coils are common at 128 MHz and below, other coils besides a birdcage coil can be used as a volume transmit coil, such as a transverse electromagnetic (TEM) coil, a phased coil array, or any other type of radio frequency coil. The housing HS typically includes a cosmetic inner liner defining the scanner bore BR.

The MRI arrangement may furthermore include a patient bed or table PT. The patient PAT is expected to lie on the table PT during imaging. The patient PAT is prepared for imaging in a preparatory phase whilst lying on the table PT outside the imager's bore BR. Once preparation has been concluded and imaging is to commence, the patient table PT is operated to introduce the patient PAT into the bore along imaging axis Z, the longitudinal axial direction of the cylindrical bore BR. The patient table PT may be motorized to facilitate such introduction.

In use, after introduction of the patient PAT into bore BR, the main magnetic field coils MG produce the main magnetic field Bo in a Z-direction which is preferably at least 3.0T, and more preferably greater than 3.0T, such as 7.0T or higher. Lower than 3T, such as 1T or 1.5 T, is also envisaged.

Broadly, operation of MRI comprises an image control stage and an image reconstruction stage RECON. Both stages may be administered by suitably configured one or more computing devices.

In the control stage, a configured computing device or system CS, such as a suitably programmed and interfaced general purpose or dedicated computing device, referred to herein as the image controller, co-ordinates the above described reception and transmission of radio frequency signals through the main coil MC, control of the gradient coil(s) GC, and activation of the magnet MG, or other.

The image reconstruction stage is administered by the same or a different a computing device (not shown) that runs the above described image reconstruction software or algorithms to process the stored k-space values into MR images in image domain for display, storage or additional processing. The control stage and the reconstruction stage may be integrated into a single computing system or device.

Turning now first to the image controller stage, this includes a magnetic resonance imaging controller or control interface CL to operate magnet controllers to selectively energize the magnetic field gradient coils GC and operates a radio frequency transmitter coupled to one or more of the radio frequency coils MC to selectively energize the main, stationary, radio frequency coil or coils MC arranged inside the bore, to cause a transmit signal to be emitted and to interact with Bo and tissue. By selectively operating the magnetic field gradient coils GC and the one or more radio frequency coils MC, magnetic resonance is generated and spatially encoded in at least a portion of a selected region of interest of the imaging subject PAT. In this manner, an imaging plane E to include at least a part of the ROI may be selected. What is proposed herein, and as will be explained in more detail below, is a facilitator system FS that facilitates data acquisition by imager IA by automatically providing such a definition of such a plane E for use by controller CL. Operation of facilitator FS will be explained in more detail below.

Generally, the magnetic resonance imaging control interface CL operates a radio frequency receiver coupled to one or more of the radio frequency coils MC to receive image data in form of magnetic resonance k-space data samples that are stored in a k-space memory. The image data may be stored in any suitable format.

The image reconstruction stage RECON may apply a suitable reconstruction algorithm such as a Fourier transform reconstruction algorithm to reconstruct the k-space samples into a reconstructed image including at least a portion of the region of interest of the imaging subject.

The reconstructed image $\varepsilon$ is stored in an image memory, displayed on a user interface of an operator console, stored in non-volatile memory, transmitted over a local intranet or the Internet, or otherwise viewed, stored, manipulated, or so forth. The user interface of the operator console can also enable a radiologist, technician, or other operator of the magnetic resonance imaging scanner IA to communicate with the magnetic resonance imaging controller IC to select, modify, and execute magnetic resonance imaging sequences.

As shown in Fig. 1, the image reconstructor RECON and the image controller CL may be coupled through a suitable communication network CN with the above described hardware and/or software components of the imager IA. The communication network CN, which may be wired or wireless, allows data flow from and to the imager IA. Control signals may be passed from the image controller CL to the imaging apparatus IA. Receive signals picked up at main coil MC may be passed through the network CN to the image controller IC and/or into storage in k-space memory and from there onwards to the image reconstructor RECON where they can be processed into imagery. Feeding the data from the coils MC directly to the image reconstructor RECON in a data feed for processing is also envisaged.

Some imaging protocols may require using additional RF coils, sometimes referred to as surface coils SC. There are coil components over and above and separate from the above described main RF coil MC or the gradient coil GC. Whilst the coil work such as the main gradient coil MC or gradient coil GC are stationary components, fixed and installed around the bore BR in the imager IA housing, the surface coils SC are mobile objects removable from or placeable into the bore with the patient PAT. More particularly, the surface coils can be used to provide a smaller more detailed field-of-view-imaging, with improved in-slice spatial resolution, and higher signal-to-noise ratio for a particular region of interest to be imaged. Such regions of interest ("ROI") may include a particular anatomy, such as head, chest, wrist knee, foot etc. When surface coils SC are called for, these may be used alongside and in addition to the main coil MC or instead thereof.

As mentioned, control of operation of the data acquisition by MRI imager AI may be through the computing system CS, to which reference is now made in yet more detail.

The computing system CS may be implemented on a single platform or device, or across multiple computing platform or devices, systems etc. The control computing system CS may be situated in the exam room, but this is not required as it can be located anywhere across the medical facilities or indeed off-site such as in cloud-based computing solution or distributed computing architectures. The computing system may be implemented on a work-station WS, or on an operator console OC, or on a combination thereof. At least part of the computing system CS functionalities may be supported or implemented by a mobile device, such as a laptop, tablet or smart phone. Some functionalities may be implemented in the Cloud, such as on one or more remote or on-site server computers. Mobile devices and servers may be operated in combination to carry some or all functionalities into practice.

Data acquisition may be controlled at least in parts, if not fully, automatically by running an imaging protocol program on the computing system CS. Alternatively, or in addition, data acquisition may be controlled manually by a user through suitable user interface of operating console OC, as required. In either case, data acquisition (also referred to herein as "imaging") control signals are issued through suitable one or more control interfaces CL in order to control operation, in particular of the gradient coils GC and/or the (main) exciter radiation frequency ("RF") coil MC (there may be more such RF coils, but we will, for simplicity, simple refer herein to the "the" RF coil MC). In particular, a current that is caused to pass through the gradient coils GS and the timing thereof is so controlled, and so are switching between transit and receive mode of the RF coil MC. Operating the gradient coils GC and the RF coil MC effects the data acquisition. From the data so acquired, one or more sectional images (possibly in different imaging planes) may be obtained by reconstruction using a suitable algorithm. Contrast in such MRI imagery corresponds, that is varies, with spatial proton (Hydrogen nuclei) distribution in the examination domain, and hence on or within the patient's body. Imaging for other odd-numbers Z elements (that have an odd number of protons and neutrons) can also be done if needed. However, imaging for Hydrogen density (its protons) is very common, and we will refer to such mainly below, but this is for illustration only, as MRI based on response signals from other elements with unpaired nuclei, such as $^{13}C$, $^{14}N$, $^{19}F$ or $^{31}P$ are also envisaged herein. Broadly, the main magnet MG causes alignment of hydrogen nuclei (protons) with its magnetic field $B_0$. The RF pulse emitted by RF coil MC disturbs this alignment. Once RF pulse is switched off, disturbed nuclei relax back into alignment thus, emitting during their relaxation, the resonance pulses which are picked up by coils MC in receive mode as the (in this case, RF) projection data.

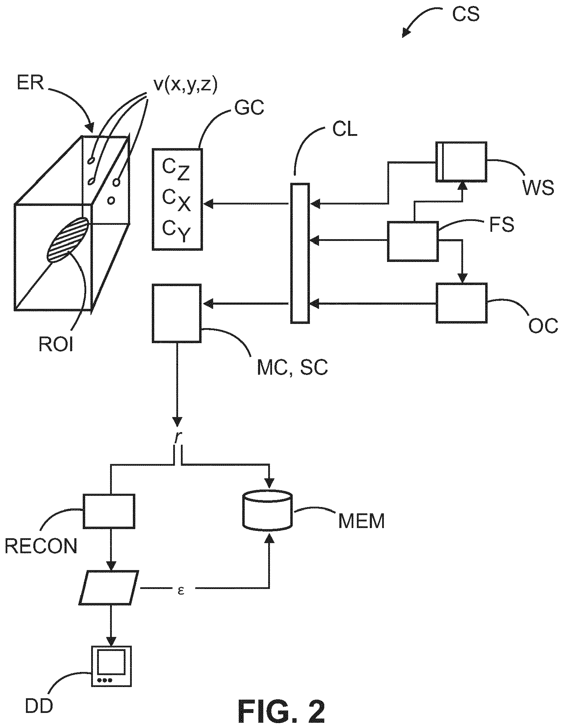

As illustrated in Fig. 2, operating the gradient coils GC allows acquiring such data for any such given imaging plane E in space ER. The gradient coils GC may comprise three sets of coils CX,CY,CZ, at least one such set for each of the spatial co-ordinate axes (X,Y,Z). The Z direction/axis is shown in Fig. 1, and is generally taken to be parallel to the longitudinal axis of the patient, and hence runs parallel to the longitudinal axis of the bore BR. The bore's lumen defines the (3D) examination region ER, also referred to herein as image domain ER (the two terms being used interchangeably herein). The remaining axis X, Y define standard planes, perpendicular to Z axis. Axis X may be referred to as the lateral or Frontal axis, whilst, perpendicular thereto, the other axis, axis Y, is commonly called the Sagittal axis.

The image domain ER is a portion of 3D space. It may be thought of as spanned by such coordinate system (X,Y,Z). The image domain ER is conceptually made up of a 3D grid of spatial points, referred to as voxels $v(x,y,z)$,

as shown in Figs. 1, 2. The reconstructed sectional imagery $\varepsilon$, in particular in the imaging plane $E = E(\varepsilon)$ thereof, is situated in image domain in any desired orientation, as prescribed by user and/or protocol, purpose of imaging, the particular view desired, etc. Thus, each imaging plane, and by extension the image it supports, defines a particular clinal view. As the patient PAT, or at least the region of interest ROI, such as the heart in cardiac MRI, or an abdominal organ, patient's head, leg, arm, or any other organ of interest, resides in the examination region ERM, the grid points $v(x,y,z)$ are also applicable to positions within the patient (in-tissue position), which are often main interests herein.

The RF coil MC is operated to issue, in transmit mode, radio frequency pulses into the examination region for interaction with protons in the human tissue resident in the image domain. In addition RF coil MC may be switched to receiver mode to receive resonance response signals from the protons in response to the pulse transmitted in transmit mode. The response signals are the data acquired during a scan operation of MRI imager IA, from which the sectional image $\varepsilon$ of the spatial distribution of the Hydrogen protons can be reconstructed by the reconstructor RECON. The gradient coils GC have a different function. The gradient coils GC are operated for spatial localization in the desired plane E of the resonance response signals caused by the RF coil MC. The gradient coils GC and RF coils are operated in concert according to a scan timing protocol. As prescribed by the imaging protocol or imaging task at hand, at suitable timing, suitable currents are applied to any one of the three sets CZ, CX, CY of the spatial gradient coils GC, and to the RF coil to cause excitation pulses.

The scan operation through the imaging domain, and hence through the region of interest, may thus be defined by suitably switching and applying respective currents to the three gradient coil sets CZ, CX, CY, in concert with the switching between transmit and receive mode of the RF coil MC. In general, one or more RF excitation pulses are issued from the RF coil MC into the examination region. After that, currents may be applied, in sequential order, to suitable ones of the spatial gradient coils CZ, CX and CY, to so localize the response signal in plane E. The so localized response signal is received at transceiver coil MC in receive mode. Many read-out protocols are envisaged herein, such as line-by-line, radial, or other. The read-out data for the plane E are the 2D Fourier transforms of the sought after proton density. Thus, the reconstructor RECON may apply an inverse 2D Fourier transform to reconstruct the image $\varepsilon$ in plane E. Other reconstruction approaches, such as iterative reconstruction, not necessarily Fourier transform based, are also envisaged herein.

The gradient coils CX, CY, CZ are operated to ensure that the RF coil MC receives resonance response signals only (or predominantly) from protons that are situated in space ER in the predefined imaging plane E. The image data may be reconstructed and assigned to voxel positions $v(x,y,z)$ in the predefined imaging plane E to thus derive the sought after sectional image $\varepsilon$. The scan can be done for multiple imaging planes $E^j$, eg, adjacent ones, or planes at different orientation, to so reconstruct an image volume if needed.

In the control operation of the scan, respective currents are applied to the three sets of coils CZ, CX, CY to define magnetic field gradients along each of the spatial axis Z,X,Y. Generally, the magnetic field gradients so caused by the gradient coils may distort the main magnetic field of magnet MG in a predictable pattern, causing the resonance response signal (in particular its frequency) to vary as a function of position, thus allowing localization.

The order in which the sets CZ, CX, CY of gradient coils GC are activated may be down to protocol and may vary based on the imaging task at hand. For example, coil CZ may be operated first, to cause magnetic field gradient along Z axis, to enable read-out of the desired z position of plane E in 3D image domain space. Coils CX and CY, in whichever order, can then be operated likewise, by applying, along the other two spatial directions X, Y, respective magnetic field gradients to read-out the $x$ and $y$ positions of for image points in the plane $E$. The $x$ position may be read-out first, followed by y, or the other way around. Phase and frequency encoding principles may be used for the acquisition of reconstructable data for the x and y coordinates of the points in plane $E$.

Depending on the imaging purpose, the ROI, etc, the requisite imaging plane E in respective of which the gradient coils GC are operated, is often not perpendicular to either one the standard axis X,Y,Z, so can run in any orientation (oblique) through the image domain ER. For example, in cardiac MRI, imaging planes E of interest include: Vertical Long Axis (VLA) view, Horizontal Long Axis (HLA) view, Mid-ventricular Short Axis (PSA) view, 4CH (four chamber) cardiac view, SA (Short Axis Stack), L2CH (Left 2 Chamber view), R2CH (Right 2 Chamber view), Chamber view (LVOT), CLVOT (Coronal Left Ventricular Outflow Tract), RVOT (Right Ventricular Outflow Tract (sagittal)), CRVOT (Coronal Right Ventricular Outflow Tract), and others.

In order to allow switching of the gradient coils GC accordingly to so define a desired localized imaging plane E according to the current imaging requirement, the plane E needs be known beforehand. In other words, imaging plane E needs to be determined/located, such as in terms of co-ordinates for example, or in any other way. Such plane E specification or definition may be done by the user manually which is cumbersome. Preferably, as envisaged herein, the intended one or more imaging plane E may be pre-computed by the computing system CS. This can be done fully automatically or with some user involvement, as required. Automatic operation is preferred, but subsequent error compensating tweaks to the computed plane, suitably supported by a user interface, preferably graphical, are also envisaged herein.

Based on the determined imaging plane E, the imaging control interface CL can translate this imaging plane E description into appropriate control signals to drive the gradient coils GC and/or the receive/transmit RF coil MC to cause a suitable pulse sequence with localization, as explained above. Once the read-out resonance response signal raw data r from within the intended imaging plane E is received, this can be stored in memory MEM, or can be passed to the reconstructor RECON to reconstruct the cross-sectional image $\varepsilon$. The reconstructed image $\varepsilon$ can be likewise stored, or may be visualized by a visualizer VIZ on the display device DD, or can be otherwise processed, used for other control or planning tasks, etc, as required.

What is proposed herein is the said facilitator system FS, as part of the computing system CS or as a standalone system (add-on, or on-demand service, etc), which allows facilitating in particular MRI data acquisition by automatically (pre-)computing the intended imaging plane E for the medical task at hand. For example, an intended view plane, such as a particular plane through the human heart for cardiac MRI or for any other types of imaging, may be computed, as required, preferably without, or with little, user input. The facilitator system FS may be integrated into an imaging planning module as supported by the work station WS, or by the operator console CS, etc.

Broadly, as is envisaged herein, the facilitator system FS is capable of computing the imaging plane E, based on a surview image *V0* of the examination region ER with the ROI in it. Thus, the surview or scout image *V0* is preferably large enough to somewhere include the region of interest ROI. For example, for cardiac MRI, patient's torso may be scanned in a preparatory scan, prior to the scanning of the imaging plane E, to obtain the surview image V0. The processing of the scout scan V0 by facilitator FS is done without prior prescription or computation of anatomic landmarks therein as was previously done. Thus, the facilitator system FS can leapfrog directly from the surview volume to a description of the intended imaging plane. No anatomic structure or anatomic landmarks need to be computed as an intermediate result. Once the plane E is found in the volume by facilitator FS, related data s(E) can then be passed on as described to the control interface CL which translates the plane data s(E) into the requisite control signals to drive, in particular, the gradient coils GC. A cumbersome prior computing of coordinates of intermediate anatomical landmarks from which the plane can may then be derived in a second step can be avoided altogether within the newly proposed imaging facilitator FS. For example, in cardiac imaging, the "double oblique plane" procedure that proceeds through flips (hence the name, double-oblique) and shifts relative to locations of landmarks of aortic annulus and first through third cusp insertion points, etc, can be avoided and is thus made obsolete, thanks to the proposed facilitator system FS. Use of the proposed from-surview-volume-to-plane-in-image-domain-leapfrogging system FS with MRI is mainly envisaged herein, but the proposed facilitator FS may also be used with benefit with other, preferably tomographic, modalities, such as in CT, OCT (optical coherence tomography), or in particular with nuclear emission imaging, such as PET or SPECT, or others still.

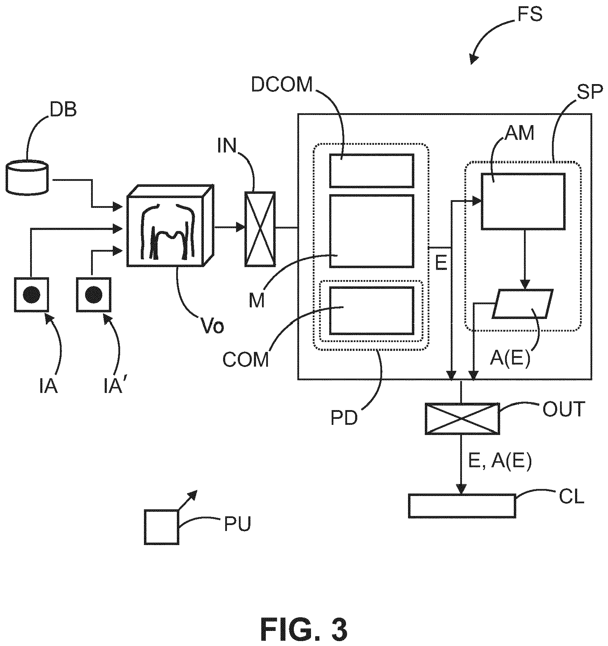

Operation of the facilitator system is now described in more detail with reference to Fig. 3. The surview image V0 is retrieved from a database or other memory where it has been stored after its acquisition, or is, directly on-the-fly as acquired, provided to the facilitator system FS through a suitable data input interface IN.

The preferably 3D surview image V0 may be an MRI surview image at a relatively low resolution, lower than intended for the subsequent acquisition in respect of the imaging plane to be computed by facilitator system FS. For example, in MRI, this surview scan image volume V0 can be done by scanning along the axis X, Y and Z, covering sufficient volume in the image domain so as to include the region of interest, such as the heart. For example, the patient's torso may be scanned, or as required in other imaging tasks, one or both of patient's arms are scanned, or a head scan is done, as required by the imaging task or purpose at hand. This surview volume V0 thus covers either the whole or a suitable part of the image domain. The gradient coils are operated to scan along all the spatial axes to so built up the surview volume. This can be done at lower resolution than the one intended for the subsequent imaging plane E-focused scan, with lower energy applied to the coils and/or can be done quicker than in the subsequent plane E based scan, thus saving time. No prior knowledge of any planes or detailed anatomical knowledge is needed when scanning for the surview (input) image V0.

The surview image V0 may be acquired prior to the subsequent plane E based diagnostic scan, with the patient still in the bore BR. However, such a "chain protocol", although preferred, is not a necessity herein. For example, the surview volume V0 may be acquired previously, in a previous session. However, it should then be ensured the patient assumes the same position in the bore BR for the subsequent plane focused scan, as was assumed previously for the surview scan. The modality for the surview scan and the subsequent plane E based diagnostic scan may even differ. For example, whilst the plane E based diagnostic scan is done with MRI scanner IA, the previous surview scan may have been done by another modality IA', such as by a CT scanner for example. This later approach may be used with benefit in a tandem system as described above, which include CT and MRI facility in one single piece of imaging equipment.

The surview image volume *V0*, gotten by whichever means, may then be image-processed by the facilitator system FS into the target imaging plane E within the said surview volume *VO.* For example, the data s(E) related to determined panel E may be provided in "ostensive definition", that is, as an explicit subset of the volume *VO* as a set of grid points that together constitute the plane E. The output as a set of points, a subset of surview volume, may be understood as one of segmentation, either binary or in fuzzy form. That is, in fizz form, per voxel probabilities measure a spatial point's membership in respect of the plane. The output may thus be represented as a mask, having the same size as surview volume V0. Alternatively, or in addition, of providing such a set of spatial grid points by ostensive definition, an analytic A(E) specification, eg in terms of a geometric locus equation, or in any other form, may be provided. The analytic A(E) specification is one other example of the mentioned plane data s(E).

Referring now in yet more detail to facilitator system FS, and with continued reference to Fig. 3, this includes a plane determiner PD that processes volume V0 into the target imaging plane E, in any of the above-mentioned or other forms. Preferably, the plane determiner RD is configured as a segmenter that computes the target imaging plane E as a sub-set of voxel points, as part of the surview volume V0. The pane determiner PD may thus also be referred to herein in embodiments as a plane segmentor PD. The so computed segmented plane as a set of spatial points v may be one or more voxels thick, so may resemble as point cloud in some cases. It is this set of spatial grid voxel points which defines the target plane E ostensively, that is, by a set that of all spatial co-ordinates of all the points that constitute the plane.

Optionally, plane determiner PD may co-process the input surview image V0 in addition to contextual data that describe the intended imaging purpose, preferably if the said purpose is not unambiguously derivable from the nature of the volume V0.

As a further option, the facilitator system FS may include a specifier SP that operates on the so segmented target imaging plane E as a sub-set to compute an alternative description such, as an analytic description A(E) of the plane E. Specifier SP may implemented as an analytical machine AM for example, such as a geometrical tool, that computes the set of points found by segmentor PD in terms of an equation, such as $\vec{n} * \vec{x} = e$ (the dot product with normal vector of generic space position vector), or as $ax+by+cz = d$, etc.

For example, analytical machine AM of specifier SP may be implemented by singular value decomposition (SVD), random sample consensus (RANSAC), or by any plane-fitting algorithm. Coefficients of the specifier A(E) of plane E may be computed by fitting to points of the point cloud, for such is the segmented plane E in some cases as observed above. More specifically, the plane fitting algorithm of analytical machine AM may use orthogonal distance regression, and/or the said singular value de-composition approach, RANSAC, etc. This and similar plane fitting setups may be configured as a minimization or maximization problem, using a cost/utility function to find the plane that is as close as possible (using a suitable distance measure) to the set of $n$ 3D guide points ($n \geq 3$) from the segmented point cloud E. For example, using a Euclidean distance function, the analytical machine AM may be configured to minimize the square sum of the orthogonal distances between the analytical plane definition A(E), and the plane segmentation E (viewed as point cloud) generated by the plane determiner PD. If more than one E plane is computed, each may be fed into the analytical machine AM for processing into its respective analytical plane definition A(E), such as coefficients for a plane equation, etc.

Such analytical specifications A(E) may be preferably in instants where the set of spatial points v as provided by plane determiner PS may be ambiguous. For example, the set of points v in input volume V0 may be more than one voxel thick, at least at places, and thus, as such, is more akin to a point cloud, albeit one in general of sufficient flatness to be aptly called a plane. Thus, the plane E as a set and as provided by plane determiner PD may be visualized in shape like a sheet or plate having a thickness of more than one voxel. However, it is expected that in most cases the point cloud is considerable thinner than its spread in the other two dimensions. Thus, the plane specifier SP may refine a potentially ambiguous set of points, a "prototype plane E", into a definite plane specification A(E). As said however, the specifier SP is optional as it may be sufficient in some instances, where there is no such ambiguity, for the control interface CL to operate direct on the sub-sets of voxel points which are produced in the segmentation operation of plane determiner PD. However, in some instances, even though the plane provided as the set by segmentor PD has no ambiguity, a translation by specifier SP into such an analytical specification A(E) may be still beneficial, for example if the inner workings of the interface logic of control interface CL so requires. Thus, in such cases the specifier SP may act like a middleware or API (application program interface).

The facilitator FS may provide a visualization on display device DD of the plane E as computed. For example, such plane E visualization may be by surface rendering or in mark-ups, overlay graphics, etc, in a visualization of the surview volume V0 and/or of the spatial coordinate system (X,Y,Z) in any desired spatial view. The facilitator FS may have its own visualizer to this effect, or may be configured to interface with the system visualizer VIZ to this end.

What is mainly envisaged in embodiments is a machine learning ("ML") based implementation of the plane determiner PD. In such embodiments, the plane determiner PD may include a trained machine learning model M, previously trained on suitable training data, as will be described in more detail below. The machine learning model may operate on the volume V0 as a whole, possibly augmented by contextual data that suitably encodes the medical imaging purpose, to produce the segmentation of the plane E in image domain ER.

For computational efficiency it may be useful to process the volume in parts, in sequence or in parallel, to so derive the segmentation result E. To this end, the plane determiner PD may include a decomposer unit DCOM, and a counterpart (re-)composition unit COM. The decomposer DCOM may receive the input surview volume V0, and define patches thereof such as 3D sub-sets, for example, sets of ellipsoids, of balls or of cubes, etc, that partition the volume. The machine learning model M may then operate on each of those patches separately, to produce a partial result for each patch. The partial results may then recomposed/assembled by the composer COM into the complete imaging plane as the final result. Thus, the patches as processed by the machine learning model may each produce a certain part of the plane E which are then together composed into a complete description of the plane E as a subset of the volume V0. Patch-wise processing via the decomposer/recomposer duo COM/DECOM may be done during training of model (on which more below), during deployment (as referred to above), or during both, training and deployment.

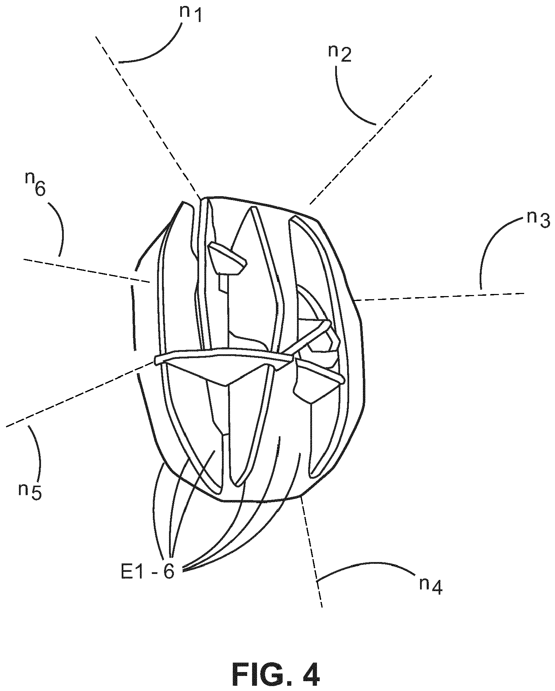

In either case, whether the optional decomposer/recomposer-duo is used or not, the imaging plane E may not necessarily extend all the way to the boundary of volume V0 (although this may still be the case in some embodiments). A sub-set of the mathematical plane that intersects the ROI may suffice, thus defining parts of the plane as a truncated plane (or "planelets"), as is illustrated below in more detail in Fig. 4 to which reference is made. Fig. 4 affords a 3D perspective view on six such planes, illustrated as planelets E1-E6, with their respective normals $n1$-$n6$. It is not necessary to compute multiple such planes as illustrated in Fig. 4, a single such plane may suffice, but computing multiple such planes may well be done in embodiments. Thus, in some embodiments, only a single plane as shown in Fig. 4 or similar is segmented for. As mentioned earlier, only parts of each plane are shown as planelets. As can be seen in the perspective 3D illustration of Fig. 4, the planes are sub-sets in the surrounding volume V0, and may have a certain thickness of one voxel or more across. The segmentation for each plane E, E1-E6 may thus be thought of as a sheet of a certain thickness, or like plates, situated in the volume V0, in the correct spatial configuration passing through the portion of space in the volume that represents the anatomy of interest (the ROI), such as the human heart. In fact, in cardiac imaging, multiple planes may be required, such as illustrated in Fig. 4. Specifically, in Fig. 4 the six planes are for views in sections for VLA, A4CH, SA, L3CH. Up to 11 planes may be used in cardiac imaging.

Optionally, the actual ROI (such as in this case the myocardium, or parts thereof) may be visualized in segmentation, graphical mark up or otherwise, in addition to the one or more planes E1-6. This visualization in 3D or in any other spatial view on display device DD, either of the one or more target planes E, E1-6 on their own, or in combination with a visualization of the ROI, may serve as a useful quality check tool for user. Once the user agrees with the computed plane(s), the data acquisition/scan can commence. The user may initiate this through the operator console OC. Autonomous imaging is also envisaged, where there is no such visualization, and the computed plane(s) E are passed on to imager control interface CL and scan is automatically initiated. The "direct" computing of the planes as envisaged herein does not preclude some reformatting or other downstream processing to ensure the plane data s(E) (eg, the spatial points that constitute the plane) can be processed by the control interface CL for data acquisition purposes, as explained above in relation to Figs. 1,2.

Thus, with the proposed facilitator system FS, instead of segmenting first for anatomic landmarks in the volume and then fitting planes later to co-ordinates of those segmented landmarks, the intended/desired imaging plane is directly computed with the proposed method from the input surview volume V0, without any reference to interim anatomic landmarks, or indeed to any anatomy at all (other than what is encoded in the surview image V0). For example, the complex landmark driven procedures of subsequent flipping of intermediate planes (such as in the above mentioned "double oblique" reformatting in cardiac imaging) or similar step-by-step approaches that proceed from "landmark to landmark" can be avoided herein and are made obsolete. This leapfrogging property makes the proposed system FS quicker, more robust, and more accurate.

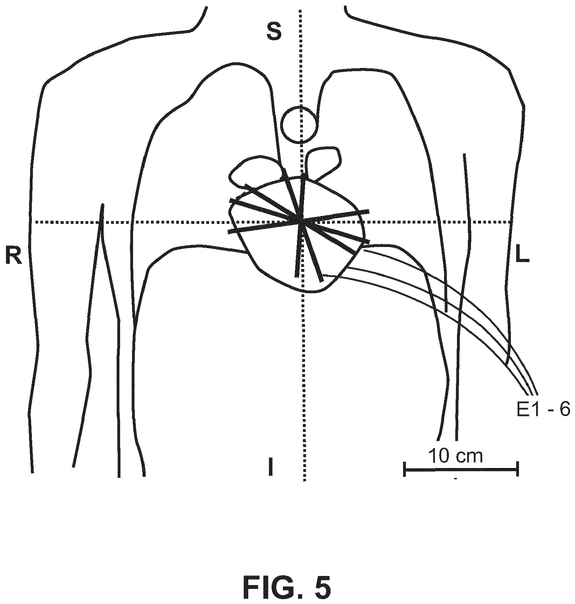

Fig. 5 is another view along one of the spatial directions (sagittal axis Y) on the imaging planes E1-E6, which now appear as lines in this view.

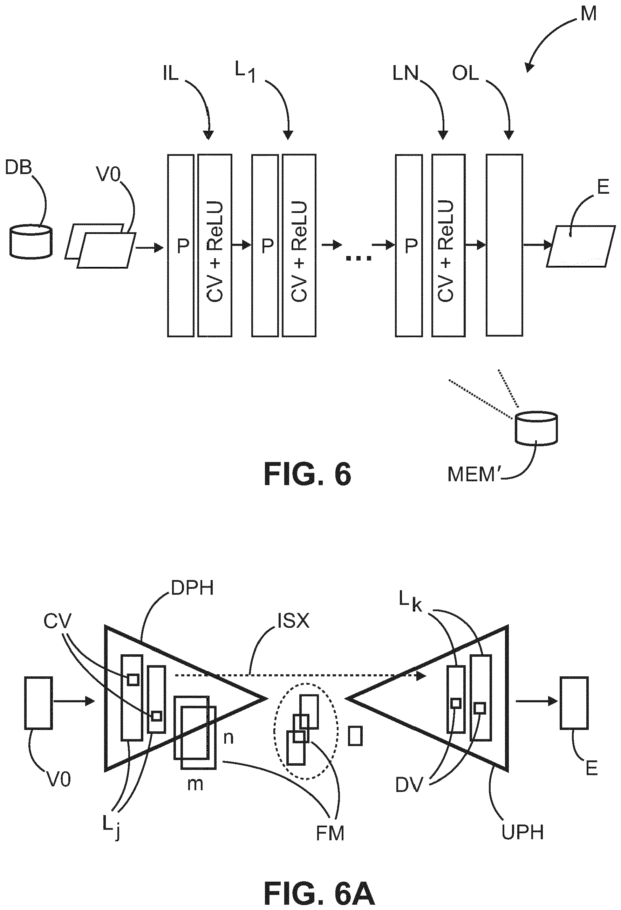

Reference is now made to Fig. 6 which explains in more detail a machine learning model M architecture as may be used by the plane determiner PD.

Machine learning models have been found to yield good results, with quick return times, especially if implemented as artificial neural network models as may be envisaged herein. In particular, artificial neural network models of the convolutional type (CNN) are envisaged herein in embodiments, as such models have been found to be well suited to process spatially correlated data such as is the surview image input data V0.

Conceptionally, there is a latent mapping between the space of input volumes V0 that encode image points globally of the region of interest, and the space of imaging planes intended as a sub-set within those volumes. This latent mapping is a function of the given image volume, and the nature of the imaging task, its medical purpose etc. This mapping may be difficult to model analytically with closed mathematical functional expressions that allow feasible evaluation. In this situation, a machine learning model can be used where a set of standard ML architectures is available, that are suitably adapted to accept input volume at a suitable size to produce the output also suitably sized. Based on a sufficiently large and varied training data set, this model can learn patterns that represent this mapping in sufficient approximation to reliably, robustly and accurately predict the segmentation of the plane based on the volume provided. The surview image encodes the ROI and also some of the surrounding anatomical landmarks. However, ML models will look at this data across the training data instances holistically during training to establish the right pattern, without any need to specifically define and compute any definition of the anatomical landmarks themselves, or indeed of even the ROI itself. The proposed model implements a more general type of segmentation. In this type of segmentation, the target structure (the plane E) itself does not need to be represented in the surview image. Instead, it is the contours of the ROI and/or the applicable landmarks allow a segmentation into the volume by taking the spatial configuration of the landmarks and the ROI only implicitly into account when placing the plane segmentation into the volume V0. Thus, the proposed system does not rely on any contour of the plane itself as there are no such contours in the volume, the plane being a conceptual a-priori-definition to support the data acquisition during imaging. Thus, the proposed processing may be thought of as segmentation "into the blind", guided by the knowledge of the imaging task intended, and the spatial distribution of possible landmarks and/or the ROI related to the imaging task.

On occasion, the machine learning model M may be capable of deducing the correct plane E merely by the surview volume provided especially if it is not a whole-body scan, but is suitably spatially focused (eg, as in CT by collimation for example) on the region of interest. However, at other times the surview volume *V0* on its own may be ambiguous, in which case it may be helpful to provide, not only the surview volume V0 as input, but also, in addition, contextual data that encodes the image purpose. For example, in cardiac imaging the contextual data c may represent/code for the view desired in medical terms, such as *VLA, HLA, PSA* etc. In such embodiment, it is the input surview volume V0 that is co-processed together with such context data c suitably coded (such as in vector or matrix format or any other) to produce the segmentation estimate of the desired imaging plane E. Context data may relate to the protocol to be used or to the ROI, etc.

As another embodiment, the model M is in fact a bank of individually trained machine learning models $M^c$ stored in one more data memories MEM', where each model $M^c$ has been trained for a different, specific image task c, respectively. Thus, upper index c may code as before for imaging purpose and/or a particular view, like the mentioned codes *"VLA"*, etc, Each of the respective model $M^c$ has been trained exclusively on training data chosen to suitably represent examples of imagery for such related imaging medical task c. In deployment, user merely need to indicate through a user interface (such as a drop-down menu or any other) as may be supported by the operator console OC for example, an indication c of the imaging purpose, the protocol or the target ROI, etc. Preferably, no deep specialist medical knowledge may be needed on the user's part, as the indication c may be provided in high-level medical terms (such as *"head scan", "cardiac scan",* etc). Upon input of the context data c by user, the system FS may select the related ML model $M^c$, and the surview image V0 is feed into the selected model $M^c$ to obtain the desired imaging plane.

With continued reference to Fig. 6, and in more detail, this shows a schematic diagram of an NN-type model, as mainly envisaged herein. Processing is arranged in various layers *Lj,* with input received at the input layer *IL.* Input layer responds with intermediate data output which is passed through the intermediate layers *Lj,* to yield further inter-hidden-layer intermediate output data, referred to as feature maps FM, that are then combined suitably by the output layer OL into a structure (such as the segmentation mask for plane E) that has the same size as the input image V0. Feature maps FM are spatial data that may be represented just like the input image as a matrix or tensor, so each feature map may be said to have size and resolution. A deep architecture may be preferred herein, which means there are usually at least one hidden layer, although their number is sometimes in 10s or 100s or even more. Feedforward models may be used, but NNs of the recursive types are not included, in which case the "depth" of the model is measured by the number of hidden layers times the number of passes. In other words, a recursive model with fewer layers than another such model, may still be said to have a deeper architecture, if there are a sufficient number of passes of recurrent processing, where feedback data is passed from downstream layer, back to upstream layer.

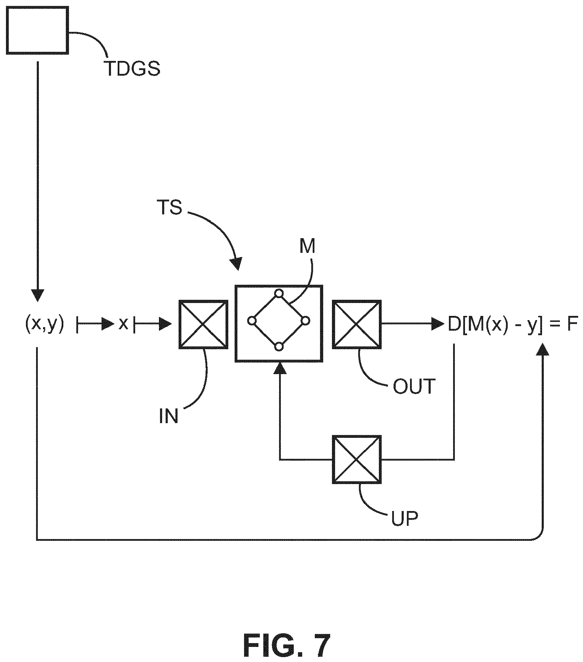

Representation, storing and processing of the feature maps FM and input/output data at input layer/output layer as matrices/ tensors affords accelerated processing. Many operations within the model can be implemented as matrix or dot-product multiplications. Thus, using matrix/tensor data representation allows some degree of parallel processing according to the single instruction multiple data ("SIMD") processing paradigm. Responsive throughput rates in training and deployment can be achieved using computing platforms with CPUs of multi-core design, such as GPUs (graphical processing units), or other computing arrangements that accommodate parallel computing. It is in particular the facilitator FS, and/or indeed the training system TS (see Fig. 7 below) which may be run with benefit on computing platforms PU with such multi-core CPUs, such a GPUs, etc. SIMD processing may help achieve efficiency gains in particular on the training system TS during training.

Preferably, some or all of the layers IL, Lj, OL are convolutional layers, that is, include one or more convolutional filters CV which process an input feature map FM from an earlier layer into intermediate output, sometimes referred to as logits. An optional bias term may be applied by addition for example. An activation operator of given layer processes in a non-linear manner the logits into a next generation feature map which is then output and passed as input to the next layer, and so forth. The activation operator may be implemented as a rectified linear unit ("RELU"), or as a *soft-max*-function, a sigmoid-function, *tanh*-function or any other suitable non-linear function. Optionally, there may be other functional layers such as pooling layers P or drop-out layers to foster more robust learning. The pooling layers P reduce dimension of output whilst drop-out layer sever connections between node from different layers.

In embodiments, the artificial neural network model (in particular of the CNN type) is adapted for multi-scale processing. Such neural networks M with multi-scale processing capability are thought to be able to cope well with the high noise and low dose surview imagery V0, where even small details can be accounted for, thus resulting in better robustness. Such CNNs configured for multi-scale processing are schematically illustrated in Fig. 6A, to which reference is now made. An example of such a convolutional neural network CNN configured for multi-scale processing is shown in the schematic diagram of Fig. 6A. Such models include, for example, the U-unit architecture or its cognates, as described by O. Ronneberger et al in "U-Net: Convolutional Networks for Biomedical Image Segmentation", available online on arXiv repository under citation code arXiv: 1505.04597 (2015).

In this or similar CNN architectures which are capable of multi-scale processing, de-convolutional operators DV may be used in some embodiments thereof. In more detail, such multi-scale enabled architecture of model M may comprise, in series, a downscale path DPH of layers and, downstream thereof, an upscale path UPH of layers. Convolutional operator with varying stride length, gradually decrease feature map size/dimension (m x n) with passage through layers Lj in downscale path, down to a lowest dimensional representation by feature maps, the latent representation $\kappa$. The latent representation feature maps $\kappa$ have their dimensions than gradually increased with passage through the layers Lk of the upscale path UPH, back to the dimension of the original input data V0. In this, the output is the plane segmentation E, represented as a mask (on which more below), so has the same size (3D) as the input surview volume V0. This dimensional bottleneck structure has been found to cause better learning as the system is forced to distil its learning down to the simplest possible structure as represented by the latent representation.

Additional regularization channels may be defined where intermediate output (intermediate feature map) from a scale level in the down-scale path fed into a corresponding layer at a corresponding scale level in the up-scale path UPH. Such inter-scale cross connections ISX been found to foster robustness and efficiency of learning and performance.

Reference is now made to Fig. 7 which shows a schematic block diagram of a training system TS that may be used to train any of the above-mentioned mentioned machine learning models M.

Broadly, training data (*x,y*) is received at the training system in order to train model M. The training data may be procured from an existing stock of medical imagery or may be synthetically generated by a training data generating system TDGS. The training data generating system TDGS may be fully automatic. Instead of synthetic generation, the training data generating system TDGS system may be configured to support annotation of existing surview imagery, for example by a human (medical) expert.

In Fig. 7, (*x,y*) represents a generic instant of training data with, x being training data input, and *y* is related ground truth or target. There are expected to be plural of such different instants of pairs of training data.

The training systems TS and the training data generator TDGS may be implemented on the same, or on different computing platforms. The platforms have preferably multicore chipsets for better throughput, in particular when it comes to implementation of the training system TS.

Turning now in more detail to training aspects, in some cases, training system TS may be controlled/driven by a cost function *F,* as illustrated in Fig. 7.

In general, two processing phases may thus be defined in relation to the machine learning models generally: a training phase and a later deployment (or inference) phase.

In training phase, prior to deployment phase, the model is trained by adapting its parameters based on the training data. Once trained, the model may be used in deployment phase to compute the alternative findings, if any. The training may be a one-off operation, or may be repeated once new training data become available.

In the training phase, an architecture of the machine learning model M, such as shown of the NN type in Fig. 6, 6A, may be pre-populated with initial set of weights. The weights $\theta$ of the model M represent a parameterization $M^\theta$, and it is the object of the training system TS to optimize, and hence adapt, the parameters $\theta$ based on the training data (*x,y*) pairs. In other words, the learning can be formulized mathematically as an optimization scheme where a cost function *F* is minimized although the dual formulation of maximizing a utility function may be used instead.

Training is the process of adapting parameters of the model based on the training data. An explicit model is not necessarily required as in some examples it is the training data itself that constitutes the mode, such as in clustering techniques or k-nearest neighbors, etc. In explicit modelling, such as in NN-based approaches and many others, the model may include as system of model functions/computational nodes, with their input and/or output it least partially interconnected. The model functions or nodes are associated with parameters $\theta$ which are adapted in training. The model functions may include the weights of the convolution operators and/or weights of the non-linear units such as a RELU, mentioned above at Fig. 6 in connection with NN-type models. In particular, in the NN-case, the parameters $\theta$ may include the weights of convolution kernels of operators CV and/or of the non-linear units. The parameterized model may be formally written as $M^\theta$. The parameter adaptation may be implemented by a numerical optimization procedure. The optimization may be iterative. The objective function F may be used to guide or control the optimization procedure. The parameters are adapted or updated so that the objective function is improved. The input training data x is applied to the model. The model responds to produce training data output M(x). The objective function is mapping from parameters space into a set of numbers. The objective function *F* measures a combined deviation between the training data outputs, and the respective targets. Parameters are iteratively adjusted to that the combined deviation decreases until a user or designer pre-set stopping condition is satisfied. The objective function may use a distance measure *D[.,.]* to quantify the deviation.

In some embodiments, but not all, the combined deviation may be implemented as a sum over some or all residues based on the training data instances/pairs, and the optimization problem in terms of the objective function may be formulated as:

$$argmin_\theta F = \sum_k D\left[M^\theta(x_k), y_k\right] \qquad (1)$$

In the setup (1), the optimization is formulated as a minimization of a cost function $F$, but is not limiting herein, as the dual formulation of maximizing a utility function may be used instead. Summation is over training data instances $i$.

The model M may be formulated as classifier model or a regression model.

The cost function $F$ may be pixel/voxel-based, such as the $L1$ - or the smoothed $L1$-norm, $L2$-norm, Hubert, or the Soft Margin cost function, etc. For example, in least squares or similar approaches, a (squared) Euclidean-distance-type cost function in (1) may be used for regression task, such as $F = \sum_k (M^\theta(x_k) - y_k)^2)$. When configuring the model as a classifier, the summation in (1) is formulated instead as one of cross-entropy or Negative Log Likelihood (NLL) divergence or similar. In some embodiments, the Dice function or the Jaccard coefficient is used as distance measure D in the cost function F, or the distance function is based or derived from Dice function or Jaccard coefficient, etc. Formulations as generative models are not excluded herein, and neither are others, including hybrids.

Updater UP of system TS updates model parameters, based on function F, in particular the gradient of $F$. The exact operation or functional composition of updater UP depends on the optimization procedure implemented. For example, an optimization scheme such as backward/forward propagation or other gradient based methods (such as gradient descent) may then be used to adapt the parameters $\theta$ of the model M so as to decrease the combined residue for all or a subset of training pairs from the full training data set. Such subsets are sometime referred to as batches, and the optimization may proceed batchwise until all of the training data set is exhausted, or until a predefined number of training data instances have been processed. The updater UP may be based on gradient descent methods, including stochastic gradient descent and cognates or others still.

Turning now in more detail to the training data generator system TDGS, this may feature a suitable user interface, to allow expert user to fashion suitably annotated training data. For example, given a training surview image, user can draw the intended plane by a graphics tool into a visualization of the plane into a visualization of the volume V, thus defining a training mask of label y for the plane. The user can use his traditional medical knowledge to draw the plane into the training volume. This expert-based training setup corresponds to supervised learning, but unsupervised learning is also envisaged herein in some embodiments, as will be briefly described below.

For example, the training input surview imagery x may be annotated by clinical experts from cardiac scan planning domain. The input training dataset {x} may include breath-hold and free-breathing whole thorax MRI imagery. The imagery may be in DICOM format. The imagery may be manually labelled. For example, 11 different cardiac view plane coordinates were used in experiments. The data may be annotated in mirador (inf format) to obtain plane annotations. Training segmentation masks y are generated from the plane annotations and passed on to training system TS to train model M. The model architecture may be as in Figs. 6,6A, described above.

In training, the model generates training data output $M(x)$ which is compared with the annotation mask y associated with the given training input surview image x. For example, the training output mask $M(x)$ mask is multiplied with the associated ground truth segmentation $y$. A distance measure D may be used to quantify the mismatch between M(x) and y. As the masks are subsets, a set theoretical distance measure, such as the Dice function or any other may be used in the cost function F to measure the set theoretic overlap between $M(x)$ and $y$. Based on the mismatch, parameters $\theta$ of the model M (such as the filter weights of the CNN model M) are adapted in one or more iterations.

The segmentation masks may be assigning or coded with unique label for each plane - for example: 1 for VLA, 2 for HLA, 3 for 3CH view, 4 for SA, 5 for 3CH view plane and 0 at the other voxel locations. The positions where the planes are overlapping, maximum index value may be taken, and assigned as the label at that location.

In embodiments, a batch size of 2 was used. Learning rate was initialized at 1e-4 and reduced if no improvement was seen for set number of epochs. Gradient based optimization may be used, as mentioned above to optimize cost function F. "Adam" algorithm, as proposed by D Kingma et al in "Adam: A Method for Stochastic Optimization", may be used, available at arXiv:1412.6980 (2014). Other stochastic gradient descent methods may be used instead.

As mentioned, Dice and/or cross entropy may be used to formulate loss function $F$. As an alternative, instead of using Dice loss for set theoretic comparison, an analytical approach is used, where the ground truth y, and the training output $M(x)$ are both transformed by analytical machine AM into respective plane equations or other such analytical definitions, using SVD or other plane fitting techniques as earlier described above in relation to deployment phase. Using Euclidean distance function D, cost function may be based on calculating angulation and/or offset differences between the computed plane M(x), and the manually annotated plane y.

As an extension, generative models such as GANs (see Ian Goodfellow et al in "Generative Adversarial Networks", available online at online repository arXiv:1406.2661, published June 2014) may be used instead to train model M to generate instance of planes E, given training data. No prior annotation is needed. For example, in GAN systems, they can proceed in a quasi-unsupervised manner by using two models pitched against each other, interlinked with their output and some input in the overall system TS. The system in this embodiment thus comprises a generator (which is the model to be trained), and a discriminator network which attempts to discriminate between instance of a plane generated by generator or instances drawn from ground truth training data set. A specifically configured cost function, based on a two-player minimax game setup is used that tends to converge to an equilibrium state thus balancing the opposed objectives of the "two players", the generator and discriminator as training proceeds over the training data instances. Generative model setups other than GANs are also envisaged herein, such as (variational) autoencoders, transformer type models, Bayesian networks, or others.

Whilst implementation as a machine learning model configured for segmentation tasks for such planes E is preferred herein, traditional non-machine learning based segmentations approaches may also be contemplated herein in alternative embodiments.

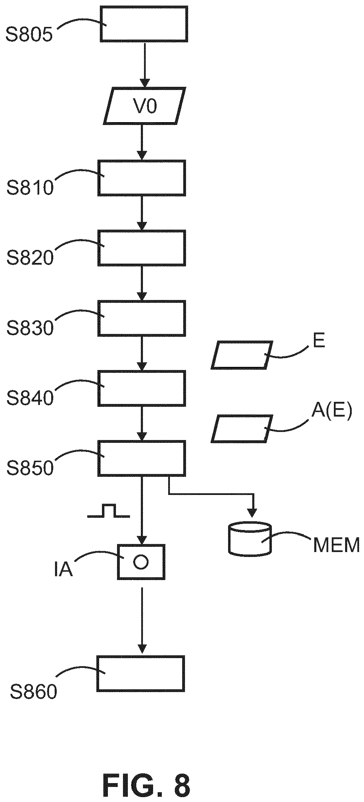

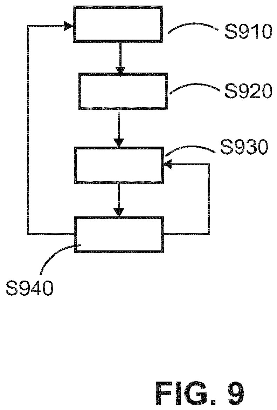

Reference is now made to the flow chart in Figs. 8 and 9 which explain aspects of the above described system FS,CS in terms of related methods. However, it will be understood that the below described method steps are not necessarily tied to such systems, but may also be understood as teachings in their own rights.

Broadly, Fig. 8 relates to deployment/inference phase, and assumes the model has already been trained, whilst Fig. 9 relates to training aspects of the model given training data.

Referring now first to employment/inference phase in Fig. 8, this represents steps of a computer implemented method for facilitating image acquisition in medical imaging, in particular for MRI. However, as mentioned earlier, restriction to MRI is not necessarily required herein as any other forms as the proposed principles can be used in any other tomographic settings including CT and emission imaging, such as SPECT and PET as required.

As an initial step S805, a preferably 3D surview image V0 is obtained, based on measurement data (such as RF data in MRI) acquired of a patient/ROI in a first data acquisition operation using a first imaging apparatus IA. Thus, the surview volume is assumed to include image information of the region of interest ROI which is the purpose of the imaging task intended in a later image-plane-based second data acquisition using the same, or a different, image apparatus IA'.

For example, the region of interest may include a human heart or part thereof. Thus, the surview volume may indeed cover at least the patient's chest. A whole-body scan may be done instead. The surview image may be obtained by reconstruction from measurement data, acquired by the same modality to be used of the subsequent image-plane-based measurement data acquisition, or the input volume V0 is based on data acquired previously by a different medical modality that also capable of providing a 3D surview image of the given patient. The surview image has, in general, a lower resolution than the image to be reconstructed from in-image domain imaging plane E, to be computed in the course of the proposed method as will be detailed below.

At step S810 the input 3D surview image volume V0, including the region of interest, obtained in whichever way, is received.

At step S820 the volume *V0* is processed, preferably by a trained machine learning model, to determine the mentioned imaging plane *E* as a subset within the input volume V0. The model is preferably trained on suitable training data. Whether or not machine learning based, the in-volume plane E is preferably determined at step S820 by segmentation, that is, as a subset of the volume V0.

The output at step S820 is preferably a description of the intended imaging plane as a sub-set of the volume. The description may be in terms of a mask. The mask may have a size (dimension $n * m * l$) equal to that of the image volume V0. The mask codes for the imaging plane E by defining within the volume spatial points v that constitute the plane. A binary mask or a probabilistic mask may be so computed in step S820. However, any other manner of providing the segmentation results of the imaging plane is envisaged herein, and the machine learning model, or any other model that may be so used, is understood herein to be suitably configured for provide a definition of the plane in a suitable format. The definition is preferably one of a plane as subset of the volume.

At step S830 the so computed segmented plane E is then output and made available for further processing. In some embodiments an additional processing step S840 may be applied, such as of specifying the plane further in terms of an analytical expression for example, or in any other way. For example, a geometric locus equation such as an equation of a plane, may be computed at step S840 based on set of spatial points (v) as provided by the segmentation step S820. This step may be advantageous if the plane as a subset produced in step S820 has a certain thickness. In order to reduce spatial ambiguity, a plane fitting algorithm based on least squares, or on any other, may be used, treating the set of points provided at step S820 as a point cloud.

At step S850 the computed plane, in whichever format, may be used to formulate control signals in order to drive data acquisition by the imaging apparatus such as the MRI imager. In addition, or instead, the segmented imaging plane may be stored in memory for later reference.

In the MRI embodiments, the signals obtained at step S850 are to drive the gradient coils at suitable currents and/or at suitable timing to so read-out in the computed plane in image domain, radio frequency resonance signals in the second data acquisition operation, different from the first data acquisition operation used to obtain the input volume V0.

In more detail, and with particular reference to MRI, scan data acquisition for surview volume V0 is distinct, and different from, the later data acquisition/scan for the sectional image in computed plane E as envisaged. The latter occurs after the former, and the spatial resolution of the latter is in general higher than the resolution of the former. Also, no predefined plane definition E is needed for the volume surview scan $V0$, as this can processed canonically, for example, slice-wise along axis Z as facilitated by gradient coil CZ, until a large enough area surely to include the ROI is covered along axis Z, with a line-by-line or radial read-out via gradient coils CX, CY for all in-slice points $x,y$ for each position z so covered. The scan for the surview volume can be done quicker and with less energy than for the subsequent image-plane focused scan S850. These observations may also hold for other tomographic imaging modalities, such as SET and SPECT/PET, etc.

The so acquired, in-plane higher resolution data may then be passed on for processing such as reconstructing S860 into sectional image ε in the imaging plane. The sectional image ε can be used for planning, visualization, storing or in any other form of processing, as required.

Reference is now made to the flow chart of Fig. 9 which shows a computer-implemented method of training a machine learning model that may be used in step S820 above.

At step S910, training data is received in the form of pairs $(x,y)$. Each pair includes an instance of a training input surview image volume $x=V0^i$, and the associated target mask for the plane segmentation $y= E^i$.

At step S920, the training input surview image x is applied to machine learning model M having current parameters, to produce a training output M(x).

A deviation, or residue, of the training output from the associated target $y$ is quantified at S930 by cost function F. One or more parameters of the model M are adapted at step S940 in one or more iterations in an inner loop to improve the cost function. For instance, the model parameters are adapted to decrease residues as measured by the cost function. The parameters include in particular weights of the convolutional operators, in case a convolutional model M is used which is indeed envisaged in embodiments.

The training method then returns in an outer loop to step S910 where the next pair of training data is fed in. In step S920, the parameters of the model are adapted so that the aggregated residues of all pairs considered are decreased, in particular minimized. The cost function quantifies the aggregated residues. Forward- backward propagation or similar gradient-based techniques may be used in the inner loop. Instead of looping over individual data training items, such looping may be done instead over sets of training data items ("batches") at once, on which more below. The selection of batches may be randomized. The parameters as set for training data in a given batch may then be used for the next batch, and the parameters θ may then be re-adjusted when optimizing for the said next batch, and so forth over all batches considered.

Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, genetic algorithms, Monte-Carlo methods, and others still.

More generally, the parameters of the model M are adjusted to improve objective function F which is either a cost function or a utility function. In embodiments, the cost function is configured to the measure the aggregated residues. In embodiments the aggregation of residues is implemented by summation over all or some residues for all pairs considered. In particular, in preferred embodiments the outer summation proceeds in batches (subset of training instances), whose summed residues are considered all at once when adjusting the parameters in the inner loop. the outer loop then proceeds to the next batch and so for until the requisite number of training data instances have been processed. Instead of processing pair by pair, the outer loop access plural pair of training data items at once and looping is batchwise. The summation over index "$k$" in eq (1) above may thus extend batchwise over the whole respective batch.

Although in the above main reference has been made to NNs models, the principles disclosed herein are not confined to NNs. For example, instead of using the NN for pre-processor PP, another approach such as Hidden Markov Models (HMM), or Sequential Dynamical Systems (SDS), among which especially Cellular Automata (CA) may be used.

Other models envisage herein include Support Vector Machines (SVM), or boosted decision trees may be used. Other models include generative models, such as GAN, or Transformer type models, as mentioned above.

Also, whilst the training method above is mainly fashioned around the concept of supervised learning, unsupervised segmentation training setups are not excluded herein and specifically envisaged herein, such as in the GAN models, or others still.

The deployment system FS and/or the training system TS may be implemented on computing platform CS with multi-core chip set for parallel computing. GPUs, TPUs or other such multi-score processors may be used with benefit herein to secure fast return rates.

The components of the systems CS, FS may be implemented as one or more software modules, run on one or more general-purpose processing units PU such as a workstation associated with the imager IA, or on a server computer associated with a group of imagers.

Alternatively, some or all components of systems FS, CS may be arranged in hardware such as a suitably programmed microcontroller or microprocessor, such an FPGA (field-programmable-gate-array) or as a hardwired IC chip, an application specific integrated circuitry (ASIC), integrated into the imaging system FS, CS. In a further embodiment still, the systems FS, CS may be implemented in both, partly in software and partly in hardware.

The different components of system FS, CS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. System (FS) for facilitating image acquisition in medical imaging, comprising:

   an input interface (IN) for receiving, when the system is in use, an input image volume of a at least a part of an object in an examination region (ER) of an imaging apparatus (IA);
   a plane segmentor (PD) capable to segment for at least one imaging plane (E) in the volume ($V0$), to so obtain a segmentation result, the said segmentation result including the said imaging plane as a subset in the volume; and
   an output interface (OUT) for providing output data in relation to the said imaging plane for controlling a data acquisition operation of the imaging apparatus.

2. The system of claim 1, comprising an imager control interface (CL) configured to process the output data into a control signal for controlling the data acquisition operation by the imaging apparatus (IA) in order to acquire data from which is obtainable an image of the object in respect of the said imaging plane (E).

3. The system of any one of preceding claims, wherein the said output data includes a specification (A(E), s(E)) of the imaging plane (E), as a subset.

4. The system of claim 3, wherein the specification includes any one of: i) a set of coordinate points, ii) an equation describing the subset as a geometrical locus.

5. The system of claim 3 or 4, a specifier (SP), capable to specify the segmented subset of the imaging plane in terms of the specification.

6. The system of claim 5, wherein the specifier (SP) is capable to specify the subset of the imaging plane based on a geometrical plane fitting algorithm.

7. The system of any one of the preceding claims, wherein the plane segmentor (PD) is based on a trained machine learning model (M).

8. System of claim 7, wherein the machine learning model is capable of multi-scale processing at different resolution levels.

9. System of any one of the preceding claims, wherein the imaging apparatus is of the magnetic resonance type.

10. A medical imaging arrangement, (MIA) comprising the system of any one of the preceding claims, and further comprising the imaging apparatus (IA) and(or a memory (MEM') on which is stored the machine learning model (M) of the system of any one preceding claims 7-9.

11. A computer-implemented method of facilitating image acquisition in medical imaging; comprising:

    receiving (S810), when the system is in use, an input image volume of a at least a part of an object in an examination region (ER) of an imaging apparatus (IA);
    segmenting (S820) for at least one imaging plane (E) in the volume (V0), to so obtain a segmentation result, the said segmentation result including the said imaging plane as a subset in the volume; and
    providing (S830) output data in relation to the said imaging plane for controlling a data acquisition operation of the imaging apparatus.

12. A training system (TS) capable of training, based on training data, the machine learning model (M) of the system of any one of preceding claims 7-9, or of the system as used in the arrangement of claim 10.

13. A computer-implemented method of training, based on training data, the machine learning model (M) of the system of any one of preceding claims 7-9.

14. A computer program element, which, when being executed by at least one processing unit (CS), is adapted to cause the processing unit (CS) to perform the method as per any one of claims 11,13.

15. At least one computer readable medium having stored thereon the program element of claim 14, or having stored thereon the machine learning model of the system of any one preceding claims 9-12.

**FIG. 1**

**FIG. 2**

**FIG. 3**

FIG. 4

**FIG. 5**

FIG. 6

FIG. 6A

**FIG. 7**

S805

V0

S810

S820

S830

E

S840

A(E)

S850

IA  ☐

MEM

S860

# FIG. 8

S910

S920

S930

S940

# FIG. 9

EP 4 455 707 A1

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 23 16 9723

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/177295 A1 (MAXIMO ANDRÉ DE ALMEIDA [BR] ET AL) 17 June 2021 (2021-06-17) * the whole document * | 1-11,14, 15 | INV. G01R33/56 A61B5/055 G06T7/10 |
| X | US 2022/156918 A1 (CHITIBOI TEODORA [US] ET AL) 19 May 2022 (2022-05-19) * the whole document * | 1-11,14, 15 | |
| X | US 2010/121175 A1 (JOLLY MARIE-PIERRE [US] ET AL) 13 May 2010 (2010-05-13) * paragraphs [0023] – [0027], [0047] – [0050] * * figure 8 * | 1-11,14, 15 | |
| X | US 2021/174939 A1 (HUANG CHAO [US] ET AL) 10 June 2021 (2021-06-10) * paragraphs [0035] – [0036]; figure 4 * | 1,11,14, 15 | |

TECHNICAL FIELDS
SEARCHED       (IPC)

G01R
A61B
G06T
G06N

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 27 October 2023 | Vanhaecke, Nicolas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04E07)

Europäisches
Patentamt

European
Patent Office

Office européen
des brevets

**INCOMPLETE SEARCH
SHEET C**

Application Number

EP 23 16 9723

**Claim(s) completely searchable:**
        1-11

**Claim(s) searched incompletely:**
        14, 15

**Claim(s) not searched:**
        12, 13

**Reason for the limitation of the search:**

Based on the response (dated 16-10-2023) to the clarification request issued on 13-10-2023, the search is conducted on the first independent claim of each category, thereby covering the subject-matter of claims 1-11, and partially 14-15.

## ANNEX TO THE EUROPEAN SEARCH REPORT
## ON EUROPEAN PATENT APPLICATION NO.

EP 23 16 9723

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2023

| Patent document cited in search report | | Publication date | Patent family member(s) | | Publication date |
|---|---|---|---|---|---|
| US 2021177295 | A1 | 17-06-2021 | CN<br>US | 112946547 A<br>2021177295 A1 | 11-06-2021<br>17-06-2021 |
| US 2022156918 | A1 | 19-05-2022 | NONE | | |
| US 2010121175 | A1 | 13-05-2010 | NONE | | |
| US 2021174939 | A1 | 10-06-2021 | CN<br>US | 113034428 A<br>2021174939 A1 | 25-06-2021<br>10-06-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2, , 6
- **O. RONNEBERGER et al.** U-Net: Convolutional Networks for Biomedical Image Segmentation. *arXiv repository under citation code arXiv: 1505.04597,* 2015
- **D KINGMA et al.** Adam: A Method for Stochastic Optimization. *arXiv:1412.6980,* 2014
- **IAN GOODFELLOW et al.** Generative Adversarial Networks. *arXiv:1406.2661,* June 2014